# EUROPEAN PATENT APPLICATION

(11) **EP 4 272 739 A1**
(43) Date of publication of application: **08.11.2023**
(21) Application number: 21915823.5
(22) Date of filing: 29.12.2021
(51) Int. Cl.: A61K 31/496, A61K 45/06, A61P 35/00, A61P 35/04

(54) **COMPOSITION FOR INHIBITING METASTASIS AND ENHANCING ANTICANCER DRUG SENSITIVITY**

(30) Priority: 30.12.2020 KR 20200187110
(71) Applicant: Korea Advanced Institute of Science and Technology, Daejeon 34141 (KR)
(72) Inventor: CHO, Kwang Hyun, Daejeon 34141 (KR); KIM, Nam Hee, Daejeon 34141 (KR); HWANG, Chae Young, Daejeon 34141 (KR); KIM, Tae Young, Daejeon 34141 (KR)
(74) Representative: Potter Clarkson
(86) International application number: PCT/KR2021/020161
(87) International publication number: WO 2022/146025

(57) **Abstract**

The present invention relates to a composition for inhibiting metastasis and enhancing anticancer drug sensitiviy, the composition removing, from cancer cells, stem cell properties and heterogeneity acquired by epithelial-to-mesenchymal transition (EMT), thereby being capable of inducing the cancer cells into a phenotype which has regained responsiveness to a chemotherapeutic agent. A combination of a p53 activator, a SMAD4 inhibitor and an ERK inhibitor of the present invention enables a mesenchymal cell phenotype to return to an epithelial cell phenotype, and also enables the ability of cancer cells to metastasize to be inhibited by overcoming the high plasticity and drug resistance of the cancer cells, and thus may be widely used in the field of cancer treatment.

## Description

### [Technical Field]

The present invention relates to a composition for inhibiting metastasis and enhancing anticancer drug sensitivity, the composition removing, from cancer cells, stem cell properties and heterogeneity acquired by epithelial-to-mesenchymal transition (EMT), thereby being capable of inducing the cancer cells into a phenotype which has regained responsiveness to a chemotherapeutic agent.

### [Background Art]

Cancer refers to a group of abnormal cells generated by continuous division and proliferation as the balance between division and death of cells is destroyed for various reasons, and is also called tumor. Generally, the cancer occurs from 100 parts or more of the body, including organs, white blood cells, bones, lymph nodes, and the like, and develops into serious symptoms through invasion into surrounding tissues and metastasis to other organs.

Epithelial-to-mesenchymal transition (EMT) is a process in which epithelial cells transform into cells with metastatic and invasive abilities, which has been considered a fundamental phenomenon of morphological development, including the development of tissues and organs, during the embryonic period in both vertebrates and invertebrates. However, with the development of science, it has been proven that EMT plays an important role even in wound healing in adults and in the formation and progression of cancer. The earliest and most important process in EMT is cadherin switching from E-cadherin to N-cadherin. E-cadherin is a membranous glycoprotein, and an extracellular region binds to E-cadherin molecules in adjacent cells to maintain intercellular adhesion, and an intracellular region binds to α-, β- and p120 cadherins to form the polarity and cytoskeleton of epithelial cells. E-cadherin is regulated by various signaling systems involved in cell proliferation and apoptosis, such as transforming growth factor beta (TGF-β), integrin, Wnt, receptor tyrosine kinase (RTK), and Notch. Among them, TGF-β acts on SMAD or phosphatidylinositol 3-kinase/serine-threonine kinase (PI3K/AKT), and it has been reported that PI3K/AKT plays an important role in activating EMT.

That is, epithelial-to-mesenchymal transition (EMT) is a key process of metastasis and drug resistance. The EMT process is promoted by various signals secreted from a tumor microenvironment. Cancer cells increase properties of stem cell ability through the EMT process and acquire resistance to drugs and heterogeneity in cancer cells.

Accordingly, technologies for inhibiting the metastasis of cancer cells and improving resistance to anticancer drugs have been studied in various ways, but many efforts are still required for research on therapeutic agents for restoring metastatic and resistant cancer cells to a phenotype with enhanced sensitivity.

### [Disclosure]

### [Technical Problem]

Therefore, the present inventors confirmed that a three-combination of activation of p53 and inhibition of SMAD4 and ERK signaling pathways may induce a change to a phenotype with enhanced responsiveness while studying, in order to enhance the responsiveness to a chemotherapeutic agent by removing both stem cell properties and heterogeneity acquired by EMT from metastatic and resistant cancer cells, and then completed the present invention.

An object of the present invention is to provide a pharmaceutical composition for inhibiting metastasis.

Another object of the present invention is to provide a pharmaceutical composition for co-administration for preventing or treating cancer.

Yet another object of the present invention is to provide a pharmaceutical composition for enhancing anticancer drug sensitivity.

Still another object of the present invention is to provide an anticancer adjuvant.

Still yet another object of the present invention is to provide a food composition for preventing or improving cancer.

Still yet another object of the present invention is to provide a method for treating cancer.

### [Technical Solution]

In order to achieve the object, the present invention provides a pharmaceutical composition for inhibiting metastasis including a p53 activator, a mothers against decapentaplegic homolog 4 (SMAD4) inhibitor, and an extracellular signal-regulated kinase (ERK) inhibitor.

In order to achieve another object, the present invention provides a pharmaceutical composition for co-administration for preventing or treating cancer, including a p53 activator, a mothers against decapentaplegic homolog 4 (SMAD4) inhibitor, and an extracellular signal-regulated kinase (ERK) inhibitor.

In order to achieve yet another object, the present invention provides a pharmaceutical composition for enhancing anticancer drug sensitivity including a p53 activator, a mothers against decapentaplegic homolog 4 (SMAD4) inhibitor, and an extracellular signal-regulated kinase (ERK) inhibitor.

In order to achieve still another object, the present invention provides an anticancer adjuvant including a p53 activator, a mothers against decapentaplegic homolog 4 (SMAD4) inhibitor, and an extracellular signal-regulated kinase (ERK) inhibitor.

In order to achieve still yet another object, the present invention provides a food composition for preventing or improving cancer including a p53 activator, a mothers against decapentaplegic homolog 4 (SMAD4) inhibitor, and an extracellular signal-regulated kinase (ERK) inhibitor.

In order to achieve still yet another object, the present invention provides a method for treating cancer including administering a p53 activator, a mothers against decapentaplegic homolog 4 (SMAD4) inhibitor, and an extracellular signal-regulated kinase (ERK) inhibitor to a subject.

### [Advantageous Effects]

According to the present invention, a combination of a p53 activator, a SMAD4 inhibitor and an ERK inhibitor enables a mesenchymal cell phenotype to return to an epithelial cell phenotype, and also enables the ability of cancer cells to metastasize to be inhibited by overcoming the high variability and drug resistance of the cancer cells, and thus may be widely used in the field of cancer treatment.

### [Description of Drawings]

FIG. 1 illustrates results of inducing an epithelial-to-mesenchymal transition (EMT) process in cancer cells using transforming growth factor beta (TGF-β), and expression changes in genes related to mesenchymal cells and proteins and genes related to epithelial cells by TGF-β.
FIG. 2 is a diagram illustrating changes in epithelial cell phenotypes and expression changes in proteins and genes related to mesenchymal cells and epithelial cells by the presence or absence of TGF-β and activation of p53 (Nutlin-3a treatment).
FIG. 3A is a diagram illustrating an EMT molecular regulatory network including EMT-related signaling pathways related to TGF-β and p53.
FIG. 3B illustrates results of confirming expression changes in phenotype-related proteins according to combined treatment of p53 activation (Nutlin-3a treatment) and SMAD4 (shS4) in a TGF-β-treated or -untreated experimental group.
FIG. 4A illustrates results of confirming the responsiveness to 5-fluorouracil (5-FU) and doxorubicin (DOX) according to p53 activation and expression inhibition of SMAD4.
FIG. 4B illustrates results of confirming expression changes in genes related to mesenchymal cell and epithelial cell phenotypes, and sternness according to p53 activation and expression inhibition of SMAD4.
FIG. 5A illustrates results of confirming expression changes in phenotype-related proteins according to combined treatment of activation of p53 (Nutlin-3a treatment) and ERK inhibition using SMAD4 (shS4) and MEK inhibitors or p53 activation (Nutlin-3a treatment) and inhibition of SMAD4 (shS4) and ERK (shERKs) in a TGF-β-treated or -untreated experimental group.
FIG. 5B illustrates results of confirming expression changes in genes related to mesenchymal cell and epithelial cell phenotypes, and sternness according to combined treatment of p53 activation (Nutlin-3a treatment) and inhibition of SMAD4 (shS4) and ERK (shERKs).
FIGS. 6A and 6B are results of confirming changes in responsiveness to 5-FU and doxorubicin according to combined treatment of p53 activation (Nutlin-3a treatment) and inhibition of SMAD4 (shS4) and ERK (shERKs).
FIGS. 6C and 6D are results of confirming changes in responsiveness to oxaliplatin (OX) and irinotecan (CPT-11) according to combined treatment of p53 activation (Nutlin-3a treatment) and inhibition of SMAD4 (shS4) and ERK (shERKs).

### [Modes of the Invention]

Hereinafter, the present invention will be described in detail.

The present invention provides a pharmaceutical composition for inhibiting metastasis including a p53 activator, a mothers against decapentaplegic homolog 4 (SMAD4) inhibitor, and an extracellular signal-regulated kinase (ERK) inhibitor.

The three-combined treatment of the p53 activator, the SMAD4 inhibitor and the ERK inhibitor of the present invention not only restores a mesenchymal cell phenotype of cancer cells to an epithelial cell phenotype, but also inhibits the ability of cancer cells to metastasize by overcoming high plasticity and drug resistance.

According to one embodiment of the present invention, in cancer cells, it was confirmed that the epithelial cell phenotype shown in cancer cells can be restored only by p53 activation in the absence of transforming growth factor beta (TGF-β), which induces EMT, but in cancer stimulated with TGF-β, the epithelial cell phenotype cannot be restored only by p53 activation, and the phenotype is converged to a hybrid phenotype that is neither epithelial cell phenotype nor mesenchymal cell phenotype.

According to another embodiment of the present invention, in order to restore the epithelial cell phenotype in EMT-induced cancer stimulated with TGF-β, a combination of p53 activation and the SMAD4 inhibitor is required, but three-combined treatment of the p53 activator, the SMAD4 inhibitor and the ERK inhibitor is most preferred, and it was confirmed that the combined treatment thereof may inhibit the expression of genes related to sternness and enhance the sensitivity to anticancer drugs.

Therefore, the target cancer of the present invention is preferably cancer in which epithelial-to-mesenchymal transition (EMT) has progressed, more preferably cancer with wild-type p53, much more preferably at least one selected from the group consisting of lung cancer, colon cancer, pancreatic cancer, liver cancer, thyroid cancer and breast cancer with wild-type p53, and even more preferably lung cancer with wild-type p53, but is not limited thereto. In addition, in the present invention, the EMT may be induced by a tumor microenvironment including TGF-β, but is not limited thereto.

In the present invention, the p53 activator is not limited thereto, but may be at least one selected from the group consisting of Nutlin-3a, RO6839921, NSC 146109 hydrochloride, RITA, Tenovin-1, HLI373, WR 0165, Idasanutlin, and YH 239-EE. Preferably, the p53 activator may be Nutlin-3a.

In addition, the SMAD4 inhibitor may be at least one selected from the group consisting of siRNA, shRNA, miRNA, ribozyme, antisense nucleic acid, DNA/RNA chimeric polynucleotide, compounds, and antibodies that target SMAD4 gene or protein, and a signaling pathway of SMAD4, and vectors expressing thereof.

In addition, the ERK inhibitor may be at least one selected from the group consisting of siRNA, shRNA, miRNA, ribozyme, antisense nucleic acid, DNA/RNA chimeric polynucleotide, compounds, and antibodies that target ERK gene or protein, and a signaling pathway of ERK, and vectors expressing thereof.

In the present invention, the "antisense nucleic acid" refers to DNA, RNA, or derivatives thereof containing a nucleic acid sequence complementary to a specific mRNA sequence, and serves to inhibit the translation to a protein of mRNA by binding to a complementary sequence in mRNA. The antisense sequence refers to a DNA or RNA sequence complementary to the mRNA of the gene and capable of binding to the mRNA, and may inhibit translation of the mRNA, translocation into the cytoplasm, maturation, or any other essential activity for overall biological functions.

In addition, the antisense nucleic acid may be modified at one or more base, sugar or backbone positions to enhance efficacy. The nucleic acid backbone may be modified with phosphorothioate, phosphotriester, methyl phosphonate, short-chain alkyl, cycloalkyl, short-chain heteroatomic, heterocyclic intersugar linkages, and the like. In addition, the antisense nucleic acid may include one or more substituted sugar moieties. The antisense nucleic acid may include modified bases. The modified bases include hypoxanthine, 6-methyladenine, 5-methylpyrimidine (particularly, 5-methylcytosine), 5-hydroxymethylcytosine (HMC), glycosyl HMC, gentobiosyl HMC, 2-aminoadenine, 2-thiouracil, 2-thiothymine, 5-bromouracil, 5-hydroxymethyluracil, 8-azaguanine, 7-deazaguanine, N6(6-aminohexyl)adenine, 2,6-diaminopurine, and the like. In addition, the antisense nucleic acid may be chemically bound with one or more moieties or conjugates that improve the activity and cell adhesion of the antisense nucleic acid. The antisense nucleic acid includes fat-soluble moieties, such as cholesterol moiety, cholesteryl moiety, cholic acid, thioether, thiocholesterol, fatty chain, phospholipid, polyamine, polyethylene glycol chain, adamantane acetic acid, palmityl moiety, octadecylamine, hexylaminocarbonyl-oxycol esterol moiety, and the like, but is not limited thereto. The antisense oligonucleotide may be synthesized *in vitro* by a conventional method to be administered *in vivo,* or may be synthesized *in vivo.*

In the present invention, "siRNA" means a nucleic acid molecule capable of mediating RNA interference or gene silencing. Since siRNA may inhibit the expression of a target gene, siRNA is provided as an efficient gene knock-down method or a gene therapy method.

The siRNA molecule of the present invention may have a structure forming a double chain in which a sense strand (a sequence corresponding to an mRNA sequence of any one gene selected from a target gene SMAD4 or ERK according to an embodiment of the present invention) and an antisense strand (a sequence complementary to the mRNA sequence) are located opposite each other, and the siRNA molecule of the present invention may have a single-stranded structure with self-complementary sense and antisense strands. Furthermore, siRNA may include a part which is not paired by mismatch (corresponding bases are not complementary), bulge (there is no corresponding base on one chain), etc. without limiting that a doublestranded RNA part pairing RNAs is completely paired. In addition, when an siRNA end structure may inhibit the expression of the target gene by an RNAi effect, either a blunt end or a cohesive end is possible, and a cohesive end structure may be both a 3'-end protruding structure and a 5'-end protruding structure.

The "shRNA" of the present invention is called small hairpin RNA or short hairpin RNA, and is used for silencing the gene by RNA interference. Usually, shRNA is introduced into a target cell using a vector. Such an shRNA hairpin structure is also cleaved by other intercellular substances to become siRNA.

According to an embodiment of the present invention, when Nutlin-3a is used as the p53 activator, shS4 is used as the SMAD4 inhibitor, shERKs are used as the ERK inhibitor, and the three types are treated to cancer stimulated with TGF-β in combination, it was confirmed that cancer cells, which had a mesenchymal cell phenotype, were restored to an epithelial cell phenotype by epithelial-to-mesenchymal transition (EMT) and overcome resistance to anticancer drugs.

Therefore, in the present invention, the SMAD4 inhibitor is preferably shRNA which is a nucleotide set forth in SEQ ID NO: 1, and the ERK inhibitor is preferably at least one selected from the group consisting of U0126, a nucleotide set forth in SEQ ID NO: 2, and a nucleotide set forth in SEQ ID NO: 3.

SMAD4 inhibitor (shS4): TACCATACAGAGAACATTGGA (SEQ ID NO: 1)

ERKs inhibitor (shERK1): CCTGAATTGTATCATCAACAT (SEQ ID NO: 2)

ERKs inhibitor (shERK2): CCATGAGGCAAGAAACTATAT (SEQ ID NO: 3)

In the present invention, the p53 activator, the SMAD4 inhibitor, and the ERK inhibitor may be administered simultaneously or sequentially with an anticancer agent.

In the present invention, the anticancer agent may include, without limitation, anticancer agents known in the art as long as the anticancer agent can enhance an effect of cancer treatment or metastasis inhibition by the combined treatment of the p53 activator, the SMAD4 inhibitor, and the ERK inhibitor, and may be at least one anticancer agent selected from the group consisting of, for example, 5-fluorouracil (5-FU), doxorubicin, oxaliplatin, irinotecan, carboplatin, paclitaxel, gemcitabine, and bortezomib.

The composition according to the present invention inhibits EMT to suppress cancer progression, thereby having excellent effects in preventing and treating cancer by inducing cancer cell death and inhibiting metastasis, and may inhibit metastasis, such as migration and invasion of cancer cells due to EMT inhibition, from the early stage.

Accordingly, the present invention provides a pharmaceutical composition for co-administration for preventing or treating cancer, including a p53 activator, a mothers against decapentaplegic homolog 4 (SMAD4) inhibitor, and an extracellular signal-regulated kinase (ERK) inhibitor.

In addition, according to another embodiment of the present invention, when the p53 activator, the SMAD4 inhibitor, and the ERK inhibitor were treated in combination in lung cancer cells stimulated with TGF-β, responsiveness to the anticancer agent was increased.

Therefore, the present invention provides a pharmaceutical composition for enhancing anticancer drug sensitivity including a p53 activator, a mothers against decapentaplegic homolog 4 (SMAD4) inhibitor, and an extracellular signal-regulated kinase (ERK) inhibitor.

The pharmaceutical composition for enhancing the anticancer drug sensitivity according to the present invention targets cancer in which epithelial-to-mesenchymal transition (EMT) has progressed.

The anticancer agent may include, without limitation, anticancer agents known in the art as long as the anticancer agent may enhance an effect of cancer treatment or metastasis inhibition by the combined treatment of the p53 activator, the SMAD4 inhibitor, and the ERK inhibitor, and may be at least one anticancer agent selected from the group consisting of, for example, 5-fluorouracil (5-FU), doxorubicin, oxaliplatin, irinotecan, carboplatin, paclitaxel, gemcitabine, and bortezomib.

In the present invention, the composition may be in the form of capsules, tablets, granules, injections, ointments, powders or beverages, and the pharmaceutical composition may target humans. The pharmaceutical composition may be formulated and used in the form of oral formulations, such as powders, granules, capsules, tablets, aqueous suspensions, external preparations, suppositories, and sterile injectable solutions according to a general method, respectively, but is not limited thereto.

The pharmaceutical composition according to the present invention may include a pharmaceutically acceptable carrier. The pharmaceutically acceptable carrier may be used with a binder, a lubricant, a disintegrant, an excipient, a solubilizer, a dispersant, a stabilizer, a suspending agent, a pigment, a flavoring, and the like during oral administration, may be mixed and used with a buffering agent, a preservative, a painless agent, a solubilizer, an isotonic agent, a stabilizer, and the like in the case of injections, and may be used with a base, an excipient, a lubricant, a preservative, and the like in the case of topical administration. The formulations of the pharmaceutical composition of the present invention may be prepared variously in combination with the pharmaceutically acceptable carrier described above. For example, for oral administration, the pharmaceutical composition may be formulated into tablets, troches, capsules, elixirs, suspensions, syrups, wafers, and the like and for injectable administration, the pharmaceutical composition may be formulated into a single dose ampoule or a multiple dose form. In addition, the pharmaceutical composition may be also formulated into solutions, suspensions, tablets, capsules, sustained release agents, and the like.

Meanwhile, examples of the carrier, the excipient, and the diluent suitable for the formulations may be used with, for example, lactose, dextrose, sucrose, sorbitol, mannitol, xylitol, erythritol, maltitol, starch, acacia gum, alginate, gelatin, calcium phosphate, calcium silicate, cellulose, methylcellulose, microcrystalline cellulose, polyvinylpyrrolidone, water, methylhydroxybenzoate, propylhydroxybenzoate, talc, magnesium stearate, mineral oils, or the like. In addition, the pharmaceutical composition may further include fillers, anti-coagulating agents, lubricants, wetting agents, flavorings, emulsifiers, preservatives and the like.

The route of administration of the pharmaceutical composition according to the present invention includes, but is not limited to, oral, intravenous, intramuscular, intraarterial, intramedullary, intrathecal, intracardiac, transdermal, subcutaneous, intraperitoneal, intranasal, intestinal, topical, sublingual or rectal. The "parenteral" includes subcutaneous, intradermal, intravenous, intramuscular, intraarticular, intrabursal, intrasternal, intrathecal, intralesional and intracranial injection or infusion techniques. The pharmaceutical composition according to the present invention may also be administered in the form of a suppository for rectal administration.

The pharmaceutical composition according to the present invention may variously vary depending on various factors including the activity of a specific active ingredient used, the age, body weight, general health, sex, diet, time of administration, route of administration, rate of excretion, drug combination, and the severity of a specific disease to be prevented or treated. The dose of the pharmaceutical composition varies according to the condition and body weight of a patient, the severity of a disease, a drug form, and a route and period of administration, but may be properly selected by those skilled in the art. Preferably, the pharmaceutical composition may be administered with an amount capable of obtaining a maximum effect with a minimum amount without side effects in consideration of all the factors, and may be administered in an effective dose of more preferably 1 to 10000 µg/weight kg/day, much more preferably 10 to 1000 mg/weight kg/day repeatedly several times a day. The dose does not limit the scope of the present invention in any aspect.

Further, the present invention provides an anticancer adjuvant including a p53 activator, a mothers against decapentaplegic homolog 4 (SMAD4) inhibitor, and an extracellular signal-regulated kinase (ERK) inhibitor.

In the present invention, the "anticancer adjuvant" refers to an agent capable of improving, increasing or enhancing an anticancer effect of an anticancer agent.

In the present invention, the anticancer adjuvant may be used as an anticancer agent or an anticancer adjuvant depending on a treatment concentration, and may enhance the sensitivity of the anticancer agent.

In the present invention, the anticancer adjuvant may be administered in combination with a known compound having an effect of preventing, improving or treating cancer.

The known compound may be at least one anticancer agent selected from the group consisting of 5-fluorouracil (5-FU), doxorubicin, oxaliplatin, irinotecan, carboplatin, paclitaxel, gemcitabine and bortezomib, but is not limited thereto.

Further, the present invention provides a food composition for preventing or improving cancer including a p53 activator, a mothers against decapentaplegic homolog 4 (SMAD4) inhibitor, and an extracellular signal-regulated kinase (ERK) inhibitor.

The food composition according to the present invention includes all forms such as functional food, nutritional supplements, health food, health functional food and food additives.

The term "health functional food" used herein refers to food manufactured and processed by using a specific ingredient for health supplementation as a raw material or extracting, concentrating, purifying, mixing, etc., a specific ingredient contained in a food raw material, and refers to food designed and processed to sufficiently exhibit, to the living body, biological control functions such as biological defense, regulation of biological rhythm, prevention and restoration of disease, etc. by the ingredient, and refers to food that can perform functions related to prevention or health restoration of disease.

The food composition according to the present invention may be prepared in various forms according to general methods known in the art.

For example, as the health food, a mixture itself of the p53 activator, the SMAD4 inhibitor and the ERK inhibitor according to the present invention may be granulated, encapsulated and powdered to be ingested or prepared in the form of tea, juice and drink to be drunk. In addition, the mixture of the p53 activator, the SMAD4 inhibitor and the ERK inhibitor according to the present invention may be mixed with known substances or active ingredients known to have an effect in treating cancer or overcoming resistance to anticancer drugs to be prepared in the form of a composition.

Further, the functional food may be prepared by adding a mixture of a SYK inhibitor and a MAPK signaling pathway inhibitor of the present invention to beverages (including alcoholic beverages), fruits and processed foods thereof (e.g., canned fruit, bottled food, jam, marmalade, etc.), fish, meat and processed foods thereof (e.g., ham, sausage, corned beef, etc.), bread and noodles (e.g., udon, buckwheat noodles, ramen, spaghetti, macaroni, etc.), fruit juice, various drinks, cookies, sweets, dairy products (e.g., butter, cheese, etc.), edible vegetable oil, margarine, vegetable protein, retort food, frozen food, various seasonings (e.g., soybean paste, soy sauce, sauce, etc.), etc.

A preferred content of the mixture of the p53 activator, the SMAD4 inhibitor and the ERK inhibitor of the present invention in the food composition of the present invention is not limited thereto, but may be, for example, 0.01 to 80 wt% of the finally prepared food, preferably 0.01 to 50 wt% of the finally prepared food.

The food composition of the present invention may contain various flavoring agents, natural carbohydrates, or the like as additional ingredients. As the above-mentioned natural carbohydrates, monosaccharides such as glucose and fructose, disaccharides such as maltose and sucrose, natural sweeteners such as dextrin and cyclodextrin, synthetic sweeteners such as saccharin and aspartame, and the like may be used. The ratio of the natural carbohydrates may be generally about 0.01 to 0.4 g, preferably about 0.02 to 0.03 g per 100 ml of the composition of the present invention.

In addition, the food composition of the present invention may include various nutrients, vitamins, electrolytes, flavoring agents, coloring agents, pectic acid and salts thereof, alginic acid and salts thereof, organic acid, a protective colloidal thickener, a pH adjusting agent, a stabilizer, a preservative, glycerin, alcohol, a carbonic acid agent used in a carbonated drink, or the like. In addition, the food composition of the present invention may include pulps for preparing natural fruit juices, fruit juice beverages and vegetable beverages. These ingredients may be used independently or in combination. The ratio of these additives is generally selected from the range of 0.01 to 0.1 parts by weight per 100 parts by weight of the composition of the present invention, but is not limited thereto.

Further, the present invention provides a method for treating cancer including administering a p53 activator, a mothers against decapentaplegic homolog 4 (SMAD4) inhibitor, and an extracellular signal-regulated kinase (ERK) inhibitor to a subject.

As used herein, the term "subject" refers to individuals having a cancer disease, preferably mammals such as horses, sheep, pigs, goats, dogs, including humans having a cancer disease, but preferably humans.

In the method of treating cancer of the present invention, the mothers against decapentaplegic homolog 4 (SMAD4) inhibitor, and the extracellular signal-regulated kinase (ERK) inhibitor may be administered simultaneously or sequentially with the anticancer agent.

The contents of the present invention will be described in more detail through the following Examples or Experimental Examples. However, the scope of the present invention is not limited only to the following Examples and Experimental Examples, and includes modifications of equivalent technical ideas.

### Example 1. Identification of EMT-related signaling pathways according to TGF-β stimulation

### 1-1. Induction of EMT in cancer cells by TGF-β treatment

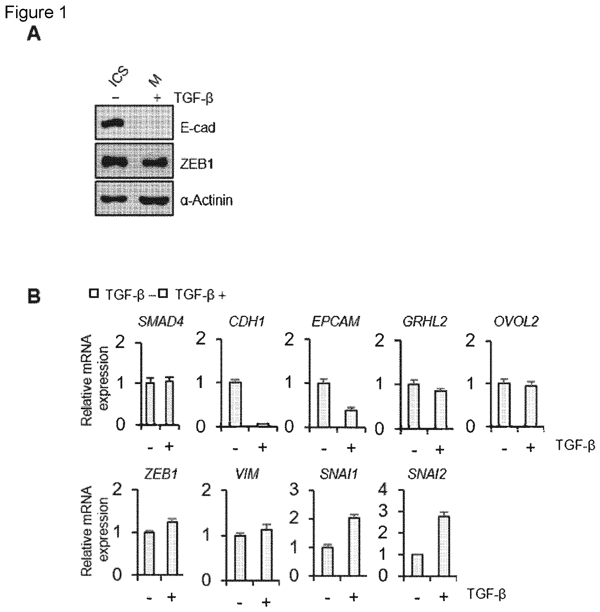

The activity of TGF-β in a tumor microenvironment performs various functions, such as promoting cancer growth and EMT. Cancer cells were treated with TGF-β, an external stimulus, to induce EMT, and the results were illustrated in FIG. 1. To this end, A549 cells (KCBL No. 10185) as a lung cancer cell line were purchased from the Korean Cell Line Bank (KCLB). EMT was induced by treating the A549 cells with TGF-β (5 ng/mL, Peprotech, Cranbury, NJ, USA) for 48 hours. Changes in protein expression of cancer cells were confirmed through western blot analysis in the following process. First, cells were lysed in a lysis buffer [including 20 mM HEPES, pH 7.2, 150 mM NaCl, 0.5% Triton X-100, 10% glycerol, protease/phosphatase inhibitor mixture (protease/phosphatase inhibitor cocktail (Thermofisher, Waltham, MA))]. The lysate was centrifuged at 13,000 rpm at 4°C for 20 minutes, and the supernatant was obtained and separated by SDS-PAGE, and then subjected to western blot analysis. To observe the amounts of proteins, E-cadherin (sc-21791), p53 (sc-126), SMAD4 (sc-7966), ZEB1 (sc-515797), mouse IgG (sc-2025) and rabbit IgG (#sc-2027) antibodies (purchased from Santa Cruz Biotechnology (Santa Cruz, CA, USA)) were used. In addition, ERK1/2 (#9102), phospho-ERK1/2 (#9106), phospho-MEK1/2 (#9121) and MYC (#13987) antibodies (Cell Signaling Technology (Beverly, MA, USA)) were used and the amounts of the proteins were observed. In addition, the rabbit polyclonal anti-GAPDH antibody was provided and used from the Korea Research Institute of Bioscience and Biotechnology.

As illustrated in A in FIG. 1, it was confirmed that since the expression of the E-cadherin protein was reduced by TGF-β treatment, a cell phenotype was changed.

In addition, qRT-PCR was performed to perform transcript analysis for all genes (n = 3, mean ± SD). The qRT-PCR analysis was performed using a PCR system (Veriti 96well Thermal Cycler, Applied Biosystems, Waltham, MA, USA), and the qRT-PCR analysis was performed using the QuantStudio 5 real-time PCR system (Applied Biosystems, Foster City, CA), samples containing 20 µL of cDNA, primers, and SYBR Master Mix (Genet Bio, Daejeon, Korea). The sequences of primers (purchased from Neoprobe, Daejeon, Korea) for this were shown in Table 1 below.

**[Table 1]**

| Target | Forward (5'→ 3') | SEQ ID NO: | Reverse (5'→ 3') | SEQ ID NO: |
|---|---|---|---|---|
| SMAD4 | CAGCCTCCCATTTCC AATCATCC | 4 | GATCTCCTCCAGAA GGGTCCAC | 5 |
| GRHL2 | GTGCCTTAGGCCTG TTGGAT | 6 | TTCTCTTGAAGGGG GAACGC | 7 |
| OVOL2 | CATGCCCAAAGTCT TCCTGGTG | 8 | ACTGGGATGTAGGT GTCTGCC | 9 |
| NCAD | GTCAGCTGTCGGTG ACAAAGC | 10 | GATCAAGTCCAGCT GCCACTGTGA | 11 |
| FN1 | CTTCTGGTCAGCAA CCCAGT | 12 | ACCGTGTGGGTACA GGTGAT | 13 |
| ZEB1 | AGCAGTGAAAGAGA AGGGAATG | 14 | TGACAGCAGTGTCT TGTTGTTG | 15 |
| MYC | GTCAAGAGGCGAAC ACACAAC | 16 | TTGGACGGACAGGA TGTATGC | 17 |
| ECAD | GAAGATTGCACCGG TCGACAAAGGACA | 18 | GCAGCTGATGGGAG GAATAACCCAGTC | 19 |
| TWIST | TCGGACAAGCTGAG CAAGATT | 20 | GCAGCTTGCCATCTT GGAGT | 21 |
| SNAI1 | GCTGCTACAAGGCC ATGT | 22 | CGGACTCTTGGTGC TTGT | 23 |
| CD90 | GAAGGTCCTCTACT TATCCGCC | 24 | TGATGCCCTCACAC TTGACCAG | 25 |
| VIM | CCAGGCAAAGCAGG AGTC | 26 | CGAAGGTGACGAGC CATT | 27 |
| b-Actin | AGAGCTACGAGCTG CCTGAC | 28 | AGCACTGTGTTGGC GTACAG | 29 |
| GAPDH | TGATGACATCAAGA AGGTGGTGAAG | 30 | TCCTTGGAGGCCAT GTGGGCCAT | 31 |

As a result, as illustrated in B in FIG. 1, it was confirmed that genes SMAD4, ZEB1, VIM, SNAI1, and SNAI2 related to mesenchymal cells were activated by TGF-β, and some GRHL2 and OVOL1 of genes related to epithelial cells did not show significant changes, but the expression of genes CDH1 and EpCAM related to representative epithelial cells was reduced. Therefore, it was confirmed that EMT of cancer cells was induced by TGF-β treatment.

### 1-2. Confirmation of restoration of epithelial cell phenotype by p53 activation and establishment of EMT molecular regulatory network

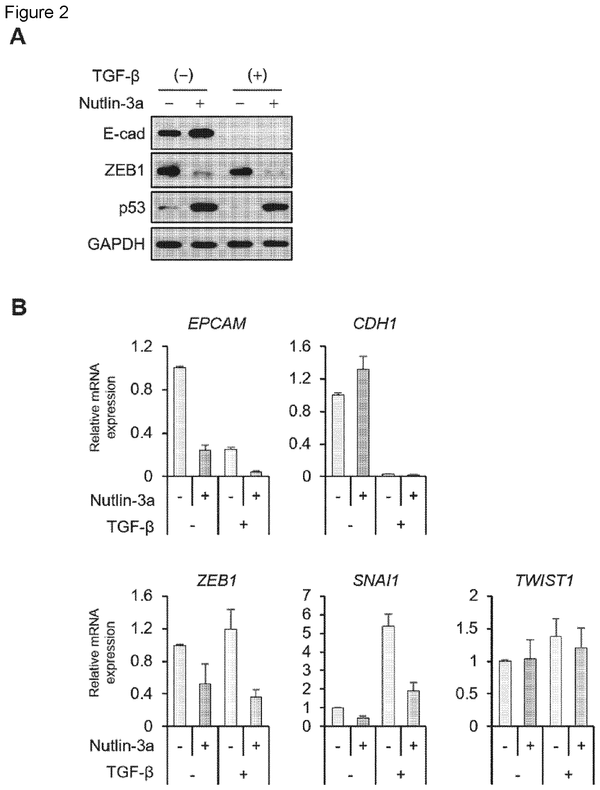

TGF-β-treated cancer cells and -untreated cancer cells were treated with Nutlin-3a (5 µM, Sigma, St. Louis, Mo) to activate p53. It was confirmed whether the epithelial cell phenotype was restored according to p53 activation, and the results were shown in FIG. 2. At this time, after inducing EMT of A549 cells under the same conditions as in Example 1-1, Nutlin-3a (1 µM, Sigma, St. Louis, Mo) was treated, and Western blot and qRT-PCR were performed in the same method as in Example 1-1.

As illustrated in A. of FIG. 2, in a TGF-β-untreated experimental group, the epithelial cell phenotype (activation of E-CAD and inactivation of ZEB1; E-CAD+/ZEB1-) was restored only by p53 activation (Nutlin-3a treatment), but in a TGF-β-treated experimental group (with TGF-β maintained), it was confirmed through changes in protein expression that when p53 was activated, the phenotype was converged to inactivation of E-CAD and inactivation of ZEB1; a E-CAD-/ZEB1-phenotype.

In addition, as illustrated in B. of FIG. 2, it was confirmed that in the TGF-β-treated experimental group, when p53 was not activated, genes ZEB1, SNAI1, and TWIST1 related to mesenchymal cells were activated, but when p53 was activated, the activity of ZEB1 was significantly inhibited. In the case of SNAI1, the activity was suppressed as compared to the TGF-β-treated experimental group, but the activity thereof was still high as compared with the TGF-β-untreated experimental group. In the case of TWIST1, it was confirmed that there was almost no change in TWIST1 activity by activating p53 in any experimental group. In addition, it was confirmed that in the TGF-β-treated experimental group, when p53 was activated, genes CDH1 and EpCAM related to epithelial cells were rather decreased or still showed the low activity.

As described above, it was confirmed that the restoration to the epithelial cell phenotype was impossible only by p53 activation in the state in which TGF-β was maintained.

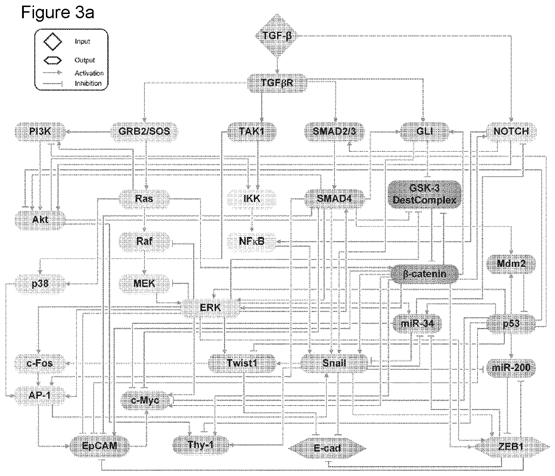

Therefore, reflecting this, in order to find the signaling pathway changed by TGF-β stimulation, and to find a combination capable of restoring the epithelial cell phenotype under TGF-β stimulation, an EMT molecular regulatory network was constructed, and the results were illustrated in FIG. 3A.

As illustrated in FIG. 3A, the EMT molecular regulatory network included EMT-related signaling pathways related with TGF-β and p53.

### Example 2. Confirmation of resistance improvement by combined regulation of SMAD4 and p53 activation

SMAD4 inhibition was derived as a target for restoring the epithelial cell phenotype (activation of E-CAD and inactivation of ZEB 1; E-CAD+/ZEB1-) through computer simulation analysis using the EMT molecular regulatory network shown in FIG. 3A.

To this end, the SMAD4 gene was first deleted in an A549 cell line. For *Lentivirus* production, HEK 293T cells were transfected for 60 hours according to the manufacturer's instructions through Lipofectamine (Thermofisher Scientific, Waltham, MA) using a vector (Sigma-Aldrich) encoding shRNA (shS4, SEQ ID NO: 1) targeting SMAD4 and packaging mix (pLP1, pLP2 and pLP/VSVG). Thereafter, the supernatant was obtained by centrifugation and filtered using a 0.22-µm filter (surfactant-free cellulose acetate, Sartorius, Goettingen, Germany). After treatment with 4 µg/ml of polybrene (Sigma-Aldrich), the A549 cells were infected. The infected cells were selected using puromycin (1 mg/ml) (Sigma-Aldrich).

As described above, the A549 cells from which the SMAD4 gene was deleted were seeded in a 60 mm dish, and after 24 hours, EMT was induced and Nutlin-3a was treated in the same manner as in Example 1-2, and then the western blot analysis was performed in the same manner as in Example 1-1.

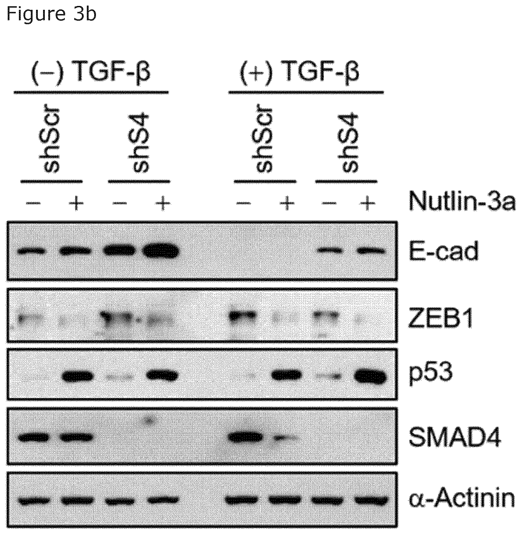

As a result, as illustrated in FIG. 3B, it was confirmed through changes in expression of proteins related to the phenotype that the epithelial cell phenotype (activation of E-CAD and inactivation of ZEB1; E-CAD+/ZEB1-) was restored through the combined treatment of p53 activation and a SMAD4 inhibitor (shS4) in the TGF-β-treated experimental group. Accordingly, it was confirmed that the epithelial cell phenotype was restored through the combined treatment of p53 activation and the SMAD4 inhibitor. Therefore, drug resistance could also be expected to be restored by such phenotypic transition (shScr in FIG. 3B means Scramble shRNA expressing cells).

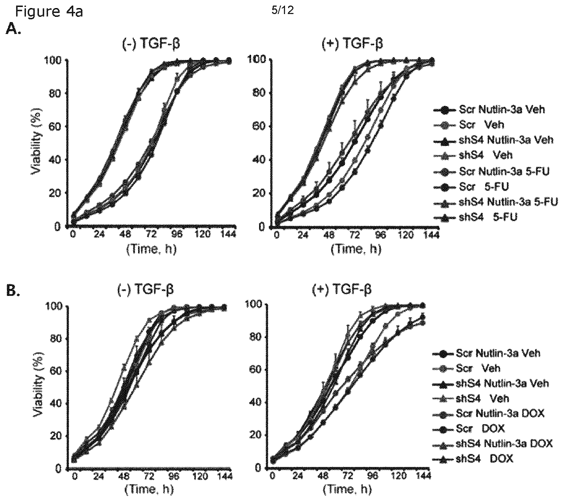

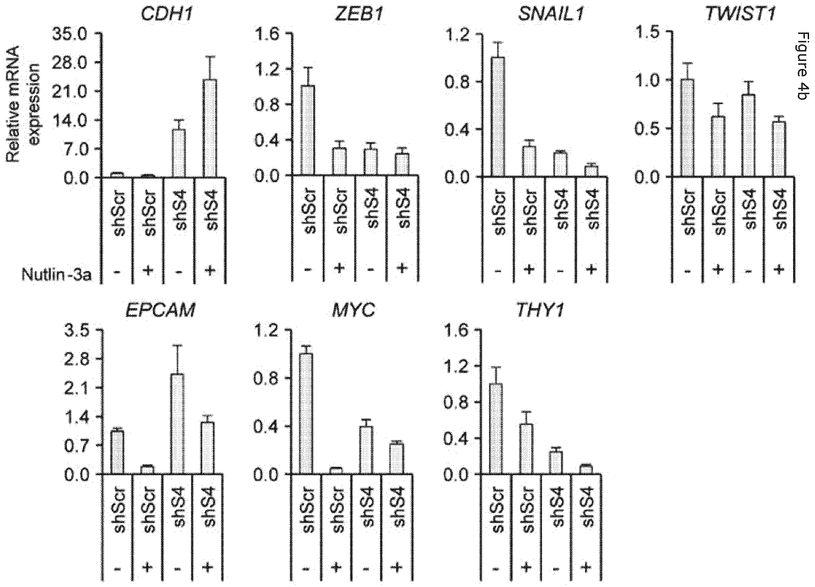

In addition, in order to measure cell viability, the A549 cells from which the SMAD4 gene was deleted as described above were seeded in a 96-well plate and after 24 hours, EMT was induced and Nutlin-3a (1 µM) was treated, and then an anticancer drug 5-FU (1 µM) or DOX (0.5 µM) was treated for 5 days. In FIG. 4A, after 5 days, the cells were imaged using IncuCyte ZOOM (Sartorius, Gottingen, Germany), and the cell viability was measured by confluence of adherent living cells using IncuCyte software. In addition, the results were confirmed by qRT-PCR in the same manner as in Example 1-1 and illustrated in FIG. 4B (n = 3, mean ± SD).

As illustrated in FIG. 4A, it was confirmed that when chemotherapeutic agents 5-fluorouracil (5-FU) and doxorubicin (DOX) were treated even in combined treatment of p53 activation and the SMAD4 inhibitor (shS4) according to Nutlin-3a treatment, cell death did not occur. As a result, it was confirmed that the epithelial cell phenotype was restored by the combined treatment of p53 activation and the SMAD4 inhibitor, but drug resistance was not completely improved.

In addition, as illustrated in FIG. 4B, it was confirmed that the epithelial cell phenotype in the ECAD+/ZEB1- state was restored through high activity of genes E-CAD related to the epithelial cells and low activity of genes SNAL1 and ZEB1 related to the mesenchymal cells. In addition, it was confirmed that genes MYC and THY1 related to sternness showed low activity. However, it was confirmed that TWIST1, a gene related to mesenchymal cells, was still activated, and the activity of EpCAM related to sternness was also high.

### Example 3. Confirmation of resistance improvement according to activation of p53 and inhibition of SMAD4 and ERK

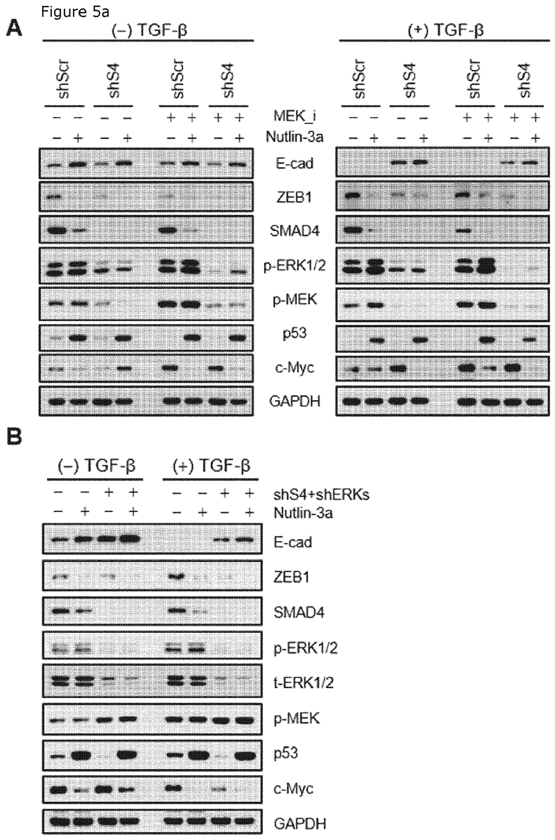

Since it was confirmed through Example 2 that the combined control of SMAD4 inhibition and p53 activation was insufficient to improve cancer cell resistance, it was confirmed whether resistance may be improved by inhibiting the ERK signaling pathway, as illustrated in FIGS. 5A to 5B.

To this end, EMT was induced in the A549 cells from which the SMAD4 gene was deleted in the same manner as in Example 2, and Nutlin-3a was treated, and an MEK inhibitor (U0126, MEK1/2 inhibitor, APExBIO, Boston, MA, USA) at a concentration of 5 µM was treated thereto.

Alternatively, in the same manner as in Example 2, the A549 cells from which the SMAD4 gene was deleted were treated with shRNAs set forth in SEQ ID NO: 2 (shERK1) and SEQ ID NO: 3 (shERK2), thereby deleting the ERK gene. Then, the results were confirmed by western blot analysis or qRT-PCR (n = 3, mean ± SD).

For the ERK gene deletion, the same method as in Example 2 was performed, and at this time, instead of a vector encoding the SMAD4-targeting shRNA, a vector (Sigma-Aldrich) encoding ERK1-targeting shRNA (shERK1, SEQ ID NO: 2) and ERK2-targeting shRNA (shERK2, SEQ ID NO: 3) was used.

As illustrated in A. of FIG. 5A, when p53 activation, SMAD4 inhibition and ERKs inhibition through the MEK inhibitor (U0126) were treated in combination in an experimental group continuously treated with TGF-β, the restoration of the epithelial cell phenotype (activation of E-CAD and inactivation of ZEB1; E-CAD+/ZEB1-) was confirmed through changes in the expression of proteins related to the phenotype.

In addition, as illustrated in B. of FIG. 5A, even if TGF-β was continuously present, the cells were restored to the E-CAD+/ZEB1- phenotype by three-combined treatment of p53 activation and inhibition of SMAD4 (shS4) and ERK (shERKs), and the tendency was also confirmed to be similar to A. in FIG. 5A.

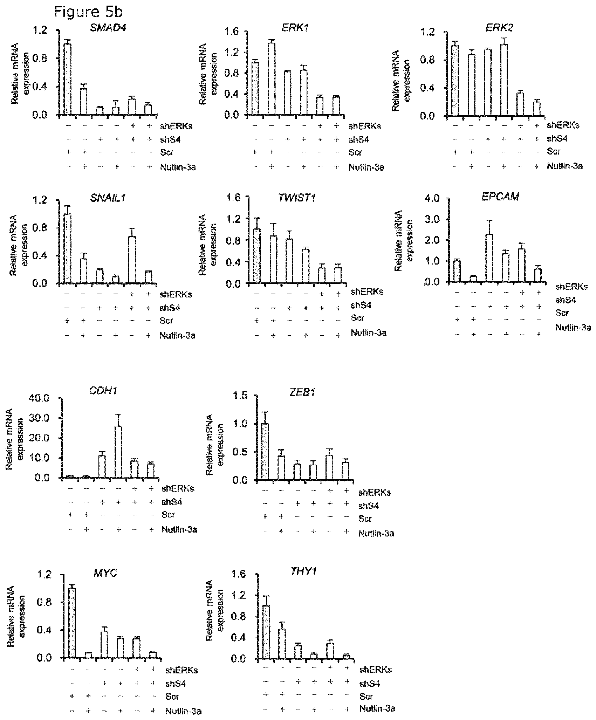

In addition, as illustrated in FIG. 5B, the expression of THY1 and MYC, which were genes related to sternness, were still inhibited, and in FIG. 4B above, it was confirmed that the expression of the gene TWIST1 related to mesenchymal cells, and EpCAM related to sternness, which had been still highly active, was inhibited through three-combined treatment of p53 activation (Nutlin-3a treatment) and inhibition of SMAD4 (shS4) and ERK (shERKs).

### Example 4. Confirmation of resistance improvement according to activation of p53 and inhibition of SMAD4 and ERK

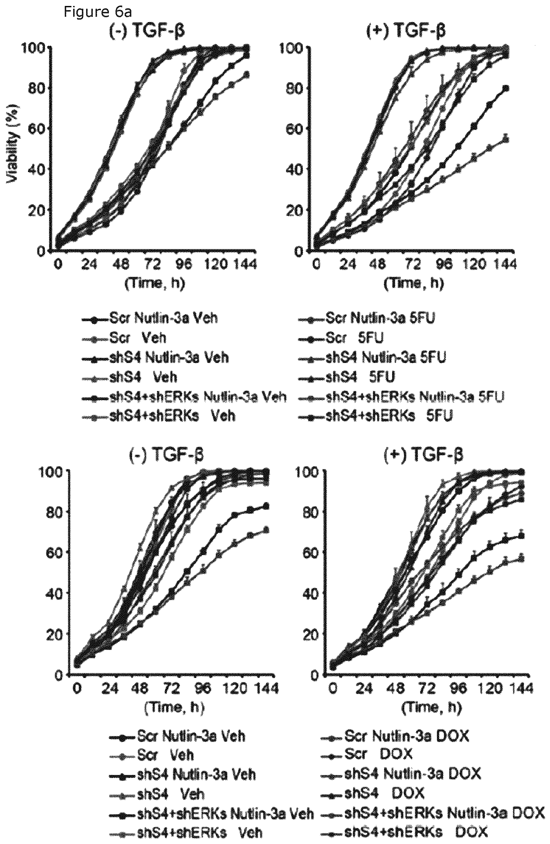

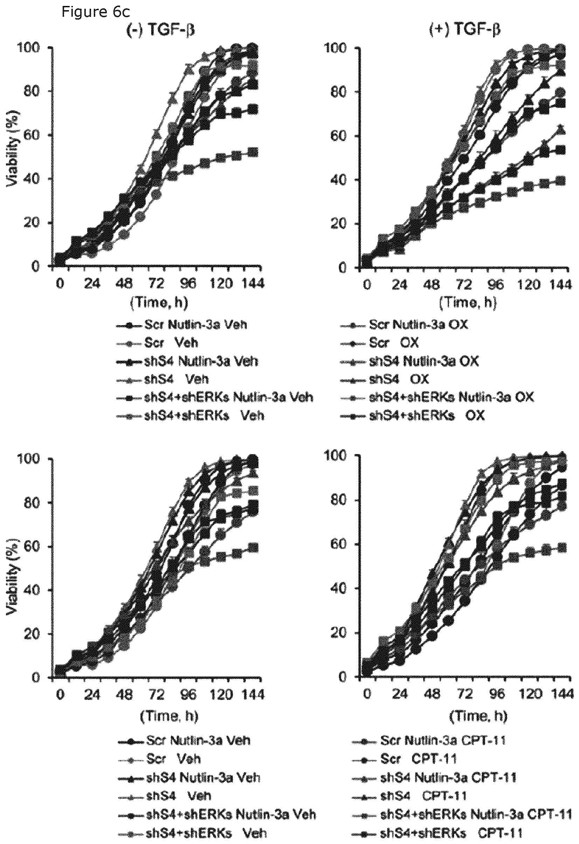

In order to confirm whether resistance was improved by the combined treatment, SMAD4 and ERK gene-deleted A549 cells were treated with TGF-β (5 ng/mL) in the same manner as in Example 3, and treated with Nutlin-3a (1 µM). In addition, 5-FU (1 µM), DOX (0.5 µM), OX (1 µM) or CPT-11 (1 µM) purchased from Sigma-Aldrich (Saint Louis, MO, USA) was treated and cultured for 5 days, respectively. Each experimental group consisted of TGF-β-treated and -untreated, and Nutulin-3a-treated and -untreated experimental groups. After 5 days, the cells were imaged using IncuCyte ZOOM (Sartorius, Gottingen, Germany), and the cell growth was measured by confluence of adherent living cells using IncuCyte software, as illustrated in FIGS. 6A and 6C.

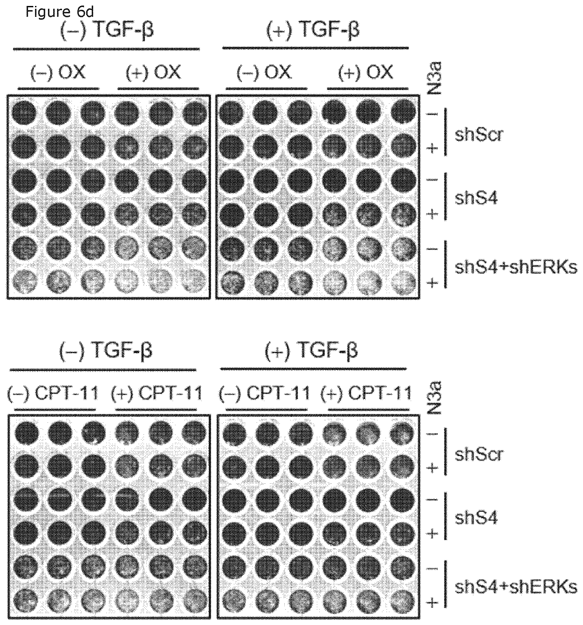

In addition, the cells were treated with each drug in combination, cultured for 5 days, and stained with crystal violet (0.5% (w/v) crystal violet (Sigma-Aldrich)) to measure cell growth, as illustrated in FIGS. 6A and 6D.

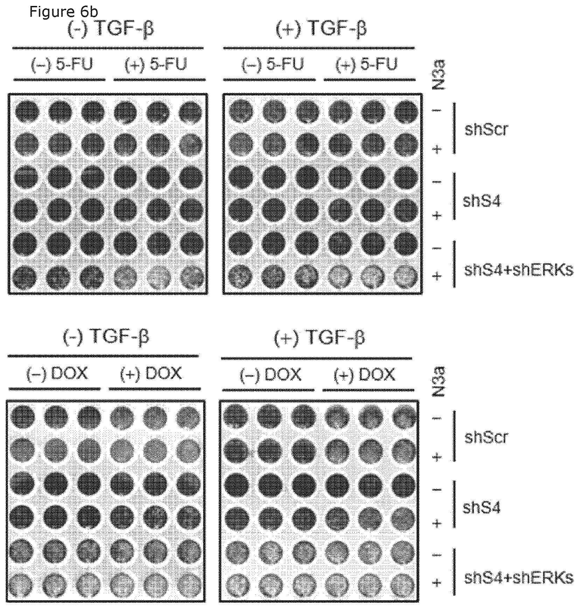

As a result, it was confirmed that the responsiveness to anticancer drugs was not changed by the combined treatment of p53 activation and the SMAD4 inhibitor (shS4), but the responsiveness to all four types of anticancer drugs was increased by combined treatment with p53 activation (Nutlin-3a (N3a) treatment) and SMAD4 (shS4) and ERK (shERKs) inhibitors. In addition, it was confirmed that cells were significantly killed by combined treatment with p53 activation (Nutlin-3a (N3a) treatment) and SMAD4 (shS4) and ERK (shERKs) inhibitors (see the last line in FIGS. 6B and 6D).

Through this, it was confirmed that the three-combined combination of activation of p53 and inhibition of SMAD4 and ERK signaling pathways could block the acquisition of metastatic ability of cancer cells in advance and overcome resistance to anticancer drugs.

Overall, according to the present invention, it is confirmed that a combination of the p53 activator, the SMAD4 inhibitor, and the ERK inhibitor removes both heterogeneity and stem cell properties acquired by EMT in cancer cells, thereby being capable of inducing the cancer cells into a phenotype which has regained responsiveness to a chemotherapeutic agent. As a result, since the combination overcomes the high plasticity and drug resistance of cancer cells and inhibits the ability of cancer cells to metastasize, it can be usefully used for cancer treatment.

## Claims

1. A pharmaceutical composition for inhibiting metastasis comprising a p53 activator, a mothers against decapentaplegic homolog 4 (SMAD4) inhibitor, and an extracellular signal-regulated kinase (ERK) inhibitor.

2. The pharmaceutical composition for inhibiting metastasis of claim 1, wherein the cancer has progressed epithelial-to-mesenchymal transition (EMT).

3. The pharmaceutical composition for inhibiting metastasis of claim 2, wherein the cancer has wild-type p53.

4. The pharmaceutical composition for inhibiting metastasis of claim 3, wherein the cancer is at least one selected from the group consisting of lung cancer, colon cancer, pancreatic cancer, liver cancer, thyroid cancer and breast cancer.

5. The pharmaceutical composition for inhibiting metastasis of claim 1, wherein the p53 activator is at least one selected from the group consisting of Nutlin-3a, RO6839921, NSC 146109 hydrochloride, RITA, Tenovin-1, HLI373, WR 0165, Idasanutlin, and YH 239-EE.

6. The pharmaceutical composition for inhibiting metastasis of claim 1, wherein the SMAD4 inhibitor is at least one selected from the group consisting of siRNA, shRNA, miRNA, ribozyme, antisense nucleic acid, DNA/RNA chimeric polynucleotide, compounds, antibodies, and vectors expressing thereof.

7. The pharmaceutical composition for inhibiting metastasis of claim 6, wherein the SMAD4 inhibitor is a shRNA of the nucleotide set forth in SEQ ID NO: 1.

8. The pharmaceutical composition for inhibiting metastasis of claim 1, wherein the ERK inhibitor is at least one selected from the group consisting of siRNA, shRNA, miRNA, ribozyme, antisense nucleic acid, DNA/RNA chimeric polynucleotide, compounds, antibodies, and vectors expressing thereof.

9. The pharmaceutical composition for inhibiting metastasis of claim 8, wherein the ERK inhibitor is at least one selected from the group consisting of U0126, a nucleotide set forth in SEQ ID NO: 2, and a nucleotide set forth in SEQ ID NO: 3.

10. The pharmaceutical composition for inhibiting metastasis of claim 1, wherein the p53 activator, the SMAD4 inhibitor, and the ERK inhibitor restore a mesenchymal cell phenotype to an epithelial cell phenotype by epithelial-to-mesenchymal transition (EMT) of cancer cells.

11. The pharmaceutical composition for inhibiting metastasis of claim 1, wherein the p53 activator, the SMAD4 inhibitor, and the ERK inhibitor are administered simultaneously or sequentially with an anticancer agent.

12. The pharmaceutical composition for inhibiting metastasis of claim 11, wherein the anticancer agent is at least one selected from the group consisting of 5-fluorouracil (5-FU), doxorubicin, oxaliplatin, irinotecan, carboplatin, paclitaxel, gemcitabine and bortezomib.

13. A pharmaceutical composition for co-administration for preventing or treating cancer, comprising a p53 activator, a mothers against decapentaplegic homolog 4 (SMAD4) inhibitor, and an extracellular signal-regulated kinase (ERK) inhibitor.

14. A pharmaceutical composition for enhancing anticancer drug sensitivity comprising a p53 activator, a mothers against decapentaplegic homolog 4 (SMAD4) inhibitor, and an extracellular signal-regulated kinase (ERK) inhibitor.

15. The pharmaceutical composition for enhancing anticancer drug sensitivity of claim 14, wherein cancer has progressed epithelial-to-mesenchymal transition (EMT).

16. The pharmaceutical composition for enhancing anticancer drug sensitivity of claim 14, wherein the anticancer agent is at least one selected from the group consisting of 5-fluorouracil (5-FU), doxorubicin, oxaliplatin, irinotecan, carboplatin, paclitaxel, gemcitabine and bortezomib.

17. An anticancer adjuvant comprising a p53 activator, a mothers against decapentaplegic homolog 4 (SMAD4) inhibitor, and an extracellular signal-regulated kinase (ERK) inhibitor.

18. A food composition for preventing or improving cancer comprising a p53 activator, a mothers against decapentaplegic homolog 4 (SMAD4) inhibitor, and an extracellular signal-regulated kinase (ERK) inhibitor.

19. A method for treating cancer comprising administering a p53 activator, a mothers against decapentaplegic homolog 4 (SMAD4) inhibitor, and an extracellular signal-regulated kinase (ERK) inhibitor to a subject.
